# EUROPÄISCHE PATENTSCHRIFT

(11) **EP 1 043 352 B1**
(45) Veröffentlichungstag und Bekanntmachung des Hinweises auf die Patenterteilung: **23.03.2005**
(21) Anmeldenummer: 00106159.7
(22) Anmeldetag: 21.03.2000
(51) Int. Cl.: C08G 63/695, C08G 63/688, A61K 7/06

(54) **Siliconmodifizierte sulfonierte Kammpolymere und Zubereitungen, insbesondere haarkosmetische Zubereitungen auf der Grundlage von solchen siliconmodifizierten sulfonierten Kammpolymeren**
Silicon modified, sulfonated comb polymers and preparations, especially hair cosmetic preparations based on such silicon modified sulfonated comb polymers
Polymères en forme de peigne modifiés au silicium et sulfonés, et préparations, en particulier préparations cosmétiques capillaires à base de tels polymères en forme de peigne modifiés au silicium et sulfonés

(30) Priorität: 07.04.1999 DE 19915612
(43) Veröffentlichungstag der Anmeldung: 11.10.2000
(73) Patentinhaber: Beiersdorf Aktiengesellschaft, 20245 Hamburg (DE)
(72) Erfinder: Koller, Andreas, Dr., 21035 Hamburg (DE); Detert, Marion, 22455 Hamburg (DE)

(56) Entgegenhaltungen:
- EP-A- 0 204 234
- WO-A-99/45055
- DATABASE WPI Section Ch, Week 198506 Derwent Publications Ltd., London, GB; Class A25, AN 1985-035383 XP002148564 & JP 59 230057 A (TOA GOSEI CHEM IND LTD), 24. Dezember 1984 (1984-12-24)

## Beschreibung

Die vorliegende Erfindung betrifft neue siliconmodifizierte sulfonierte Kammpolymere und Zubereitungen, solche siliconmodifizierten sulfonierte Kammpolymere enthaltend. Insbesondere betrifft die vorliegende Erfindung haarkosmetische Wirkstoffe und Zubereitungen zur Festigung, Formgebung, Kräftigung und Strukturverbesserung der Haare.

Eine ansprechend aussehende Frisur wird heute allgemein als unverzichtbarer Teil eines gepflegten Äußeren angesehen. Dabei gelten aufgrund aktueller Modeströmungen immer wieder Frisuren als chic, die sich bei vielen Haartypen nur unter Verwendung bestimmter festigender Wirkstoffe aufbauen bzw. für einen längeren Zeitraum aufrechterhalten lassen.

Aus diesen Gründen werden seit geraumer Zeit teils Haarpflegekosmetika verwendet, welche dazu bestimmt sind, nach Einwirken aus dem Haar wieder ausgespült zu werden, teils solche, welche auf dem Haar verbleiben sollen. Letztere können so formuliert werden, daß sie nicht nur der Pflege des einzelnen Haars dienen, sondern auch das Aussehen der Haartracht insgesamt verbessern, beispielsweise dadurch, daß sie dem Haar mehr Fülle verleihen, die Haartracht über einen längeren Zeitraum fixieren oder seine Frisierbarkeit verbessern.

Die Eigenschaft der Fülle wird einer Frisur beispielsweise zugeschrieben, wenn das Haar nach der Behandlung nicht flach auf der Kopfhaut aufliegt und gut frisierbar ist.

Die Eigenschaft des Volumen wird einer Frisur beispielsweise zugeschrieben, wenn das Haar nach der Behandlung Fülle und Sprungkraft aufweist.

Die Eigenschaft des Body's wird einer Frisur beispielsweise zugeschrieben, wenn das Haarvolumen selbst unter äußeren, störenden Einflüssen groß bleibt.

Festigende Wirkstoffe, bei denen es sich in der Regel um polymere Verbindungen handelt, können in übliche Haarreinigungs- oder -konditioniermittel eingearbeitet werden. In vielen Fällen ist es aber vorteilhaft, sie in Form spezieller Mittel wie Haarfestiger oder Haarsprays anzuwenden.

Es gibt nun in jüngster Zeit eine Reihe von Entwicklungen auf dem Haarkosmetikgebiet, die einen Bedarf an neuartigen festigenden Wirkstoffen bzw. neuen Formulierungsformen geweckt haben. Viele dieser Entwicklungen beruhen dabei nicht auf anwendungstechnischen Nachteilen oder Unzulanglichkeiten der bekannten Mittel, sondern z.B. auf Umweltschutz-Gesichtspunkten, gesetzlichen Auflagen oder anderen "nichttechnischen" Ursachen.

So wird insbesondere verstärkt ein Übergang von Mitteln auf Basis flüchtiger organischer Verbindungen (sogenannter volatile organic compounds" oder auch kurz: VOC's), z.B. Alkoholen, zu Mitteln auf waßriger Basis angestrebt.

Der Stand der Technik läßt es aber an Wirkstoffen (Polymeren) und Zubereitungen mangeln, welche den vorab genannten Anforderungen entsprechen. Die Haartracht fixierende Zubereitungen des Standes der Technik enthalten beispielsweise in der Regel Bestandteile (synthetische oder natürliche Polymere), welche Gefahr laufen, bei teilweisen oder vollständigen Ersatz leichtfluchtiger organische Bestandteile durch Wasser eine signifikante Beeinträchtigung der Produkteigenschaften zu erfahren, was oft durch geschickte Formulierung kompensiert werden muß. Zudem zeichnen sich die fixierenden Zubereitungen des Standes der Technik haufig durch nur schwierig bzw. aufwendig zu formulierende Rezepturbestandtelle mit ungenügender Langzeitstabilität aus, wobei dieses besonders auf Siliconderivate zutrifft, die zur Verbesserung der Flexibilität und Taktilität der Polymerfilmoberfläche eingesetzt werden.

Es bestand also die Aufgabe, entsprechende Mittel zu entwickeln, die hinsichtlich der anwendungstechnischen Eigenschaften, beispielsweise dem Sprühverhalten und der Trocknungszeit bei Haarsprays, die vom Verbraucher gesteckten Erwartungen erfüllen und gleichzeitig einen reduzierten Anteil an flüchtigen organischen Verbindungen aufweisen, ohne daß die elementaren Eigenschaften des Polymerfilms auf den Haaren, wie z.B. Klarheit/Tranparenz, Oberflächentaktilität, Glanz, Elastizität und Auswaschbarkeit negativ beeinflußt werden und die Verarbeitbarkeit der Formulierungsbestandteile einfach und unproblematisch ist.

Es wurde nun gefunden, und darin liegt die Lösung der Aufgaben begründet, daß Haarbehandlungsmittel mit einem wirksamen Gehalt an einem oder mehreren wasserlöslichen und/oder wasserdispergierbare silionmodifizierte Kammpolymeren, welche gewählt werden aus der Gruppe der Polyester folgender generischer Strukturformeln 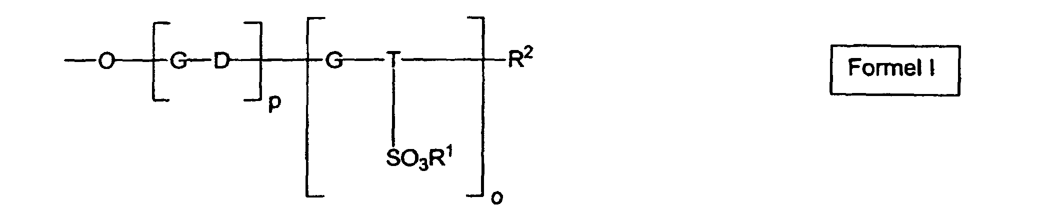 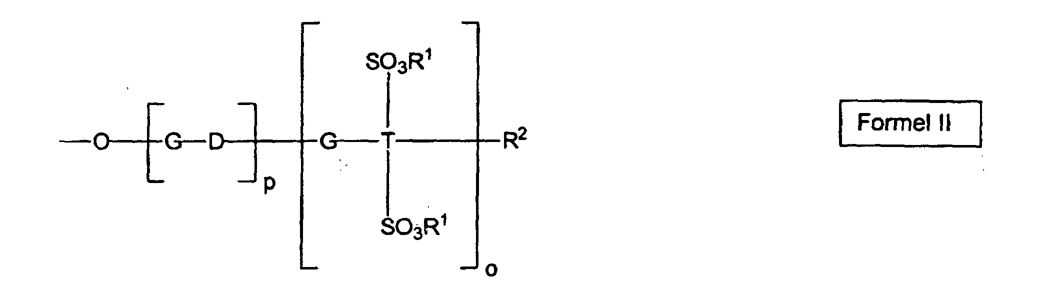 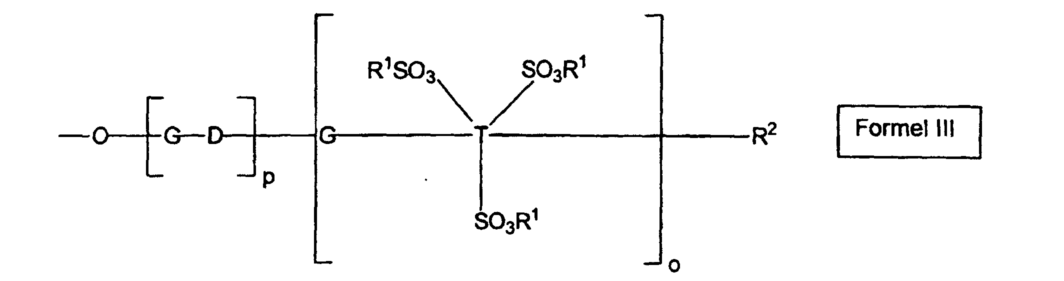 usw.
wobei p und m so gewählt werden, daß mittlere Molekulargewichte der eingesetzen Hauptkettenbestandteile zwischen 200 und 2.000.000 g/mol liegen, wobei der Bereich von 2.000 - 100.000 g/mol bevorzugt Verwendung findet.
die Polyester-Seitenketten gemäß Formel I - III bestehen aus:
- G :: einer mindestens zwei endständige Sauerstoffatome enthaltende Siloxaneinheit, die vorteilhaft durch Strukturelemente charakterisiert ist wie folgt: 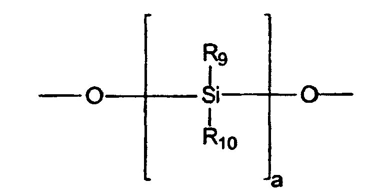 wobei die Siliciumatome mit gleichen oder unterschiedlichen Alkylresten und/oder Arylresten und/oder Arylakylresten substituiert werden können, welche hier verallgemeinernd durch die Reste R₉ - R₁₀ dargestellt sind (will sagen, daß die Anzahl der unterschiedlichen Reste nicht notwendig auf bis zu 2 beschränkt ist). a kann dabei vorteilhaft Werte von 1 - 5.000 annehmen,
sowie der mindestens zwei endständige Sauerstoffatome enthaltende aromatischen, aliphatischen oder cycloaliphatischen Organyleinheiten mit einer Kohlenstoffzahl von C₂ bis C₂₂ oder Abkömmlinge eines Polyglykols der Form HO-[R³-O)ₖ-[R⁴-O)ₘ-H, entsprechend einer Organyleinheit 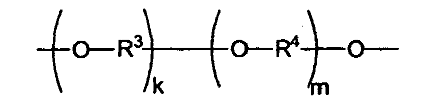 wobei die Reste R³ und R⁴ Alkylenreste darstellen mit einer Kohlenstoffzahl von C₂-C₂₂, wobei beide Reste nicht notwendigerwiese verschieden sein müssen.
wobei für die Koëffzienten k und m gilt k+m ≥ 1, wobei k und m ferner so gewählt werden können, daß die vorab bezeichneten mittleren Molekulargewichte der eingesetzen Hauptkettenbestandteite zuwege kommen.
- D:: einer mindestens zwei endständige Acylgruppen entahltenden aromatischen, aliphatischen oder cycloaliphatischen Organyleinheit mit einer Kohlenstoffzahl von C₂ bis C₂₂, wobei auch Kombinationen aus mehreren verschiedenen Säurekomponenten im beanspruchten Zielmolekül enthalten sein können, beispielsweise eine Organyleinheit des Schemas

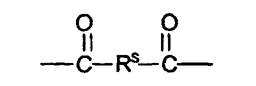

- T:: eine Verbindung aus der Gruppe der mindestens zwei endständige Acylgruppen enthaltenden sulfonierten aromatischen, aliphatischen oder cycloaliphatischen Organylverbindungen
- R¹:: Lithium, Natrium, Kalium, Magnesium, Calcium, Ammonium, Monoalkylammonium, Dialkylammonium, Trialkylammonium oder Tetraalkylammonium bedeuten kann, worin die Alkylpositionen der Amine unabhängig voneinander mit C₁ bis C₂₂-Alkytresten und 0 bis 3 Hydroxylgruppen besetzt sind.
- R² :: einen Molekülrest, gewählt aus den Gruppen der
- über Etherfunktionen verbrückenden monofunktionell-linearen oder -verzweigten siliconhaltigen Organylreste,
- aromatischen, aliphatischen oder cycloaliphatischen Aminofunktionen: ( -NH-R⁵, -NR⁵₂ wobei R⁵ einen Alkyl- oder Arylrest mit C₁ bis C₂₂ darstellen kann)
- aromatischen, aliphatischen oder cycloaliphatischen Monocarbonsäuregruppen: (-COOR⁸ wobei R⁶ ein Alkyl- oder Arylrest darstellt mit C₁ bis C₂₀₀)
- über Etherfunktionen verbrückten aromatischen, aliphatischen oder cycloaliphatischen Organylreste: (-O-R⁵)
- über Etherfunktionen verbrückenden Polyalkoxyverbindungen der Form

   -O-[R⁷-O]_{q}-[R⁸-O]ᵣ-Y

   Die Reste R⁷ und R⁸ stellen vorteilhaft Alkylenreste dar mit einer Kohlenstoffzahl von C₂-C₂₂, wobei beide Reste nicht notwendigerweise verschieden sein müssen. Der Rest Y kann sowohl Wasserstoff als auch aliphatischer Natur mit C₁-C₂₂ sein. Für die Koeffizienten q und r gilt: q+r ≥ 1.
- über Etherfunktionen verbrückenden einfach oder mehrfach ethoxylierten sulfonierten Organylreste oder bevorzugt deren Alkali- oder Erdalkalisalze, wie beispielsweise vorteilhaft gekennzeichnet durch die generische Strukturformel

   -(O-CH₂-CH₂)ₛ-SO₃R¹

   mit s ≥ 1, und wobei s ferner so gewählt werden kann, daß die vorab bezeichneten mittleren Molekulargewichte der eingesetzen Hauptkettenbestandteile zuwege kommen.
- Siliconfunktionen, die sich von monofunktionalen Siliconen ableiten gemäß der generischen Struktformel 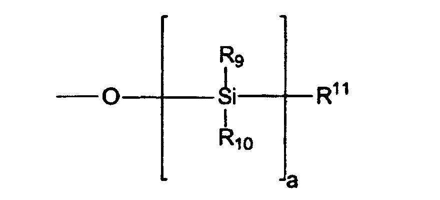
wobei R⁹ und R¹⁰ die genannten Eigenschaften haben, und unabhängi davon R¹¹ ebenfalls einen Alkylrest oder einen Arylreste oder einen Aryalkylrest darstellen kann. die Nachteile des Standes der Technik beseitigen oder zumindest mindern.

Die Dokumente WO 99745055, EP 204 234 und JP 59/230057 konnten nicht den Weg zur vorliegenden Erfindung weisen.

Die erfindungsgemäßen Kammpolymere zeichnen sich sowohl durch gute Wasser- und Alkoholverträgüchkeit als auch durch günstige Filmeigenschaften und hohem Netzvermögen aus. Zudem sind sie einfach zu formulieren.

Die siliconhaltigen Bestandteile des Polymers sind vorteilhaft in einem Anteil von 0,1 bis 50 mol-% zugegen, bezogen auf die Molmasse der Siloxaneinheit G sowie auf die Gesamtmolmasse der erfindungsgemäßen Kammpolymere. Die siliconhaltigen Anteile können unterschiedlicher chemischer Natur sein. Die beiden folgenden Typen von siliconhaltigen Komponenten werden als vorteilhafte Ausführungsformen der vorliegenden Erfindung angesehen:
a) Einerseits können lineare mindestens monofunktionelle, siliconhaltige Strukturen in die Polyesterkette mit einkondensiert werden.
   Der Einsatz von monofunktionell-linearen oder -verzweigten siliconhaltigen Derivaten während der Polykondensation führt zur Endverkappung der Polyesterketten (entspricht R² in Formel I).
   Der Einsatz von Siliconkomponenten mit 2 reaktiven Gruppen führt zu linearen Polyesterstrukturen. Der Einsatz von Siliconkomponenten mit 3 oder noch mehr reaktiven Gruppen kann zu verzweigten oder vernetzten Strukturen führen.
   Der Siliciumanteil in den Gruppierungen 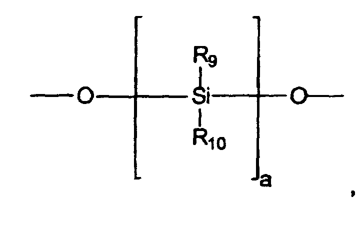 liegt vorteilhaft zwischen 0,1 und 50 mol-%. Die mittleren Molekulargewichte liegen bevorzugt zwischen 100 und 100.000 g/mol, wobei der Bereich für monofunktionelle siliconhaltige Derivate zwischen 100 und 2.000 g/mol bzw. für mindestens difunktionelle siliconhaltige Derivate zwischen 100 und 30.000 g/mol besonders bevorzugt wird.
b) Andererseits können mindestens monofunktionelle, lineare oder verzweigte, siliconhaltige Derivate auch in die polymere Hauptkette eingebaut werden.
   Auch bei diesen Derivaten liegt der Siliciumanteil vorteilhaft zwischen 1 und 50 mol-%. Die mittleren Molekulargewichte liegen bevorzugt zwischen 100 und 100.000 g/mol, wobei der Bereich zwischen 100 und 30.000 g/mol besonders bevorzugt wird.
   Bevorzugt verwendet werden dabei die Ester von Acryl- bzw. Methacrylsäure und siliciumhaltigen Monoalkoholen.
c) Selbstverständlich können auch beliebige Kombinationen jeweils einer oder mehrerer der in a) und b) beschriebenen Verbindungsklassen miteinander verwendet werden.

Die mittleren Molekulargewichte der erfindungsgemäßen Kammpolymere können vorteilhaft zwischen 200 und 2.000.000 g/mol liegen, besonders vorteilhaft zwischen 200 und 100.000 g/mol liegen wobei der Bereich von 1.000 - 30.000 g/mol bevorzugt Verwendung findet, ganz besonders vorteilhaft von 5.000 - 15.000 g/mol.

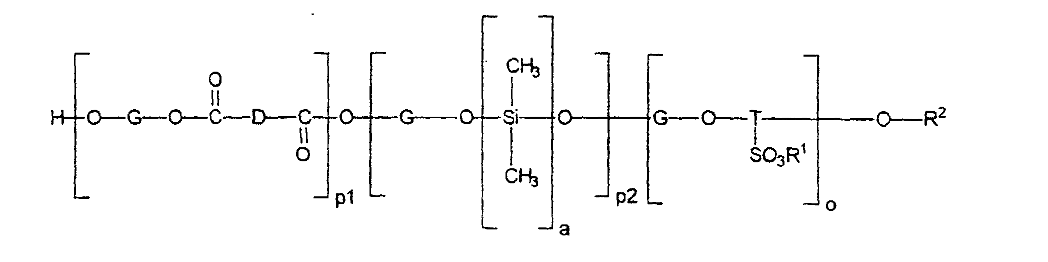

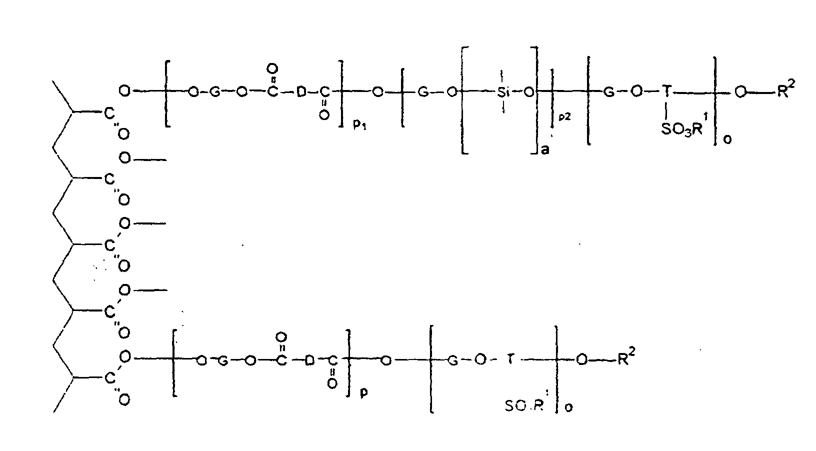

Die erfindungsgemäßen Polyester werden vorteilhaft hergestellt durch Veresterung oder Umesterung der zugrundeliegenden funktionellen Alkoholkomponenten und Drolen mit den Carbonsäuren bzw. deren geeigneten Derivate (beispielsweise Alkylester, Halogenide und dergleichen mehr) in Gegenwart eines Veresterungskatalysators wie Alkalimetallhydroxide, deren -carbonate und Acetate. Erdalkalimetalloxide, -hydroxide carbonate und -acetate sowie Alkalimetall- und Erdalkalimetallsalze von Fettsäuren mit 6 bis 22 Kohlenstoffatomen. Weiterhin kommen Titanverbindungen, wie Titanate, metallisches Zinn und organische Zinnverbindungen, wie Mono- und Dialkylzinnderivate als Veresterungskatalysatoren in Betracht. Vorzugsweise wird die Veresterung/Umesterung unter Verwendung von Zinnschliff oder Titantetraisopropylat als Katalysator durchgeführt.

Die Veresterung/Umesterung wird bevorzugt bei Temperaturen von 120 °C bis 280 °C durchgeführt, wobei der entstehende leichter siedende Kondensat (Alkohole oder Wasser) destillativ aus dem Kondensationsprodukt entfernt wird, bevorzugt unter vermindertem Druck bis zu < 0,1 mbar.

Als Edukte für die polymere Hauptkette erfindungsgemäßer, silikonmodifizierter Kammpolymere können polymere aliphatische, cycloaliphatische oder aromatische Polycarbonsäuren bzw. deren Derivate wie beispielsweise Polyacrylsäure, Polymethacryl7säure und deren Ester (Ester der beiden Säuren mit aliphatischen, cycloaliphatischen oder aromatischen Alkoholen mit C₁ bis C₂₂), Maleinsäure, Maleinsäureanhydrid, Fumarsäure und Polynorbomensäure eingesetzt werden. Die mittleren Molekulargewichte der einzelnen Polycarbonsäuren können zwischen 200 und 2.000.000 g/mol liegen, wobei der Bereich von 2.000 -100.000 g/mol bevorzugt Verwendung findet.

Auch statistische oder blockartige Copolymere der oben genannten Verbindungsklassen mit anderen vinylischen Monomeren wie beispielsweise Styrol, Acrylamid, α-Methylstyrol, Styrol, N-Vinylpyrrolidon, N-Vinylcaprolacton, Acrylamidopropylensulfonsäure und deren Alkal-, Erdalkali- und Ammoniumsalze, MAPTAC (Methacrylamidopropyltrimethylammoniumchlorid), DADMAC, Vinylsulfonsäure, Vinylphosphonsäure, Crotonsäure, Vinylacetamid, Vinylmethylacetamid, Vinylforamid, Acrylsäure oder Methacrylsäurederivate (beispielsweise freie Säure oder Ester), siliciumhaltige Acrylat- Methacrylat- oder Acrylamidderivate oder Vinylacetat können zur Ausbildung der polymeren Hauptkette dienen.

Als. Basis für mindestens zwei endständige Sauerstoffatome enthaltende aromatische, aliphatische oder cycloaliphatische Organyleinheiten mit einer Kohlenstoffzahl von C₂ bis C₂₂ oder Abkömmlinge eines Polyglykols der Form HO-[R³-O)ₖ-[R⁴-O)ₘ-H, können bifunktionelle Alkoholkomponenten eingesetzt werden.

Dafür eignen sich insbesondere mindestens difunktionelle aromatische, aliphatische oder cycloaliphatische Alkohole mit einer Kohlenstoffzahl von C₂ bis C₂₂ oder ein Polyglycol der Form HO-[R³-O]ₖ-[R⁴-O]ₘ-H. Die Reste R³ und R⁴ stellen Alkylreste dar mit einer Kohlenstoffzahl von C₂ bis C₂₂, wobei beide Reste gleich oder verschieden sein können. Für die Koeffizienten k und m gilt: k+m ≥ 1 , wobei k und m ferner so gewählt werden können, daß die vorab bezeichneten mittleren Molekulargewichte der eingesetzten Hauptkettenbestandteile zuwege kommen.

Es kann von besonderem Vorteil sein, statt difunktioneller Alkoholkomponenten tri-, tetra- oder allgemein polyfunktionelle Alkoholkomponenten einzusetzen, beispielsweise vorteilhaft gewählt aus der folgenden Gruppe:

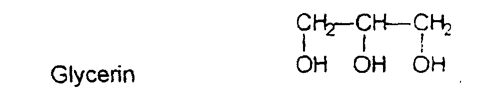

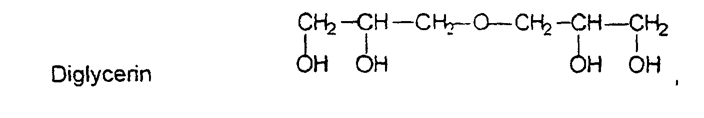

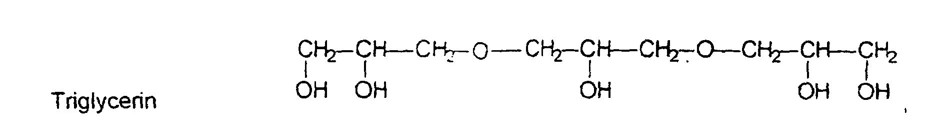

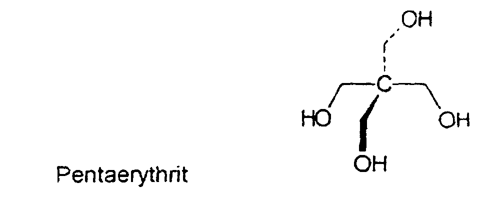

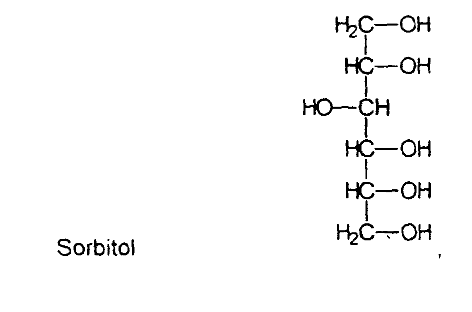

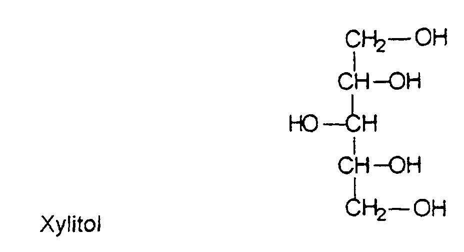

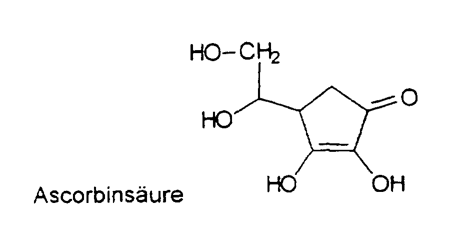

Als Basis für mindestens zwei endständige Acylgruppen enthaltende aromatische, aliphatische oder cycloaliphatische Organyleinheiten mit einer Kohlenstoffzahl von C₂ bis C₂₂, beispielsweise Organyleinheiten des Schemas 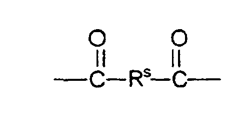 können beispielsweise aromatische und lineare oder cyclische, gesättigte oder ungesättigte aliphatische Carbonsäuren mit einer Kohlenstoffzahl von C₂ bis C₂₂ oder dessen Anhydride eingesetzt werden, beispielsweise Phthalsäure, Isophthalsäure, Naphthalindicarbonsäure, Cyclohexandicarbonsäure, Adipinsäure, Bernsteinsäure, Glutarsäure, Pimelinsäure, Korksäure, Azelainsäure, Sebacinsäure, Brassylsäure eingesetzt werden. Auch Kombinationen aus mehreren verschiedenen Säurekomponenten sind als Monomereinheit im beanspruchten Zielmolekül möglich.

Als sulfongruppenhaltige Monomere eignen sich sulfonierte aromatische, aliphatische oder cycloaliphatische Dialkohole, Disäuren bzw deren Ester, Anhydride oder Halogenide, wie beispielsweise Sulfobernsteinsäure, 5-Sulfoisophthalsäure oder deren Alkalioder Erdalkalisalze oder Mono-, Di-, Tri- oder Tetraalkylammoniumsalze mit C₁ bis C₂₂-Alkylresten. Unter den Alkalisalzen sind insbesondere Lithium- und Natriumsalze bevorzugt.

Weiterhin kommen aromatische, aliphatische oder cycloaliphatische Amine mit C₁ bis C₂₂ Alkyl- bzw Arylresten und/oder aromatische, aliphatische oder cycloaliphatische Monocarbonsäuren mit C₁ bis C₂₀₀ Alkyl- oder Arylresten und/oder Polyalkoxyverbindungen der Form -O-[R⁷-O]_{q}-[R⁸-O]ᵣ-X, wobei die Reste R⁷ und R⁸ Alkylreste, die gleich oder verschieden sein können eine Kohlenstoffzahl von C₂ bis C₂₂ darstellen und der Rest X sowohl Wasserstoff als auch aliphatischer Natur mit C₁ -C₂₂ sein kann und die Koeffizienten q und r : q+r ≥ 1 sind, zum Einsatz.

Ebenso geeignet sind sulfonierte Mono- oder Polyethylenglykole oder bevorzugt deren Alkali- oder Erdalkalisalze: (H-(O-CH₂-CH₂)ₛ-SO₃R¹ mit s ≥ 1 wobei s ferner so gewählt werden kann, daß die vorab bezeichneten mittleren Molekulargewichte der eingesetzen Hauptkettenbestandteile zuwege kommen.)

Als geeignete siliconhaltige Derivate sind beispielsweise lineare Siloxandiole, Hydroxyalkyl-endmodifizierte Siloxane, unterschiedlich hoch ethoxylierte oder propoxylierte Silanole oder verzweigte Derivate mit unterschiedlicher Funktionalität verwendbar. Solche werden im allgemeinen durch Strukturelemente charakterisiert wie folgt: 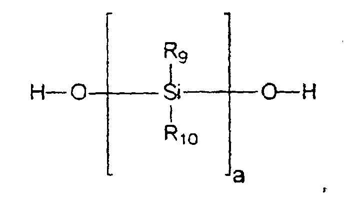 wobei die Siliciumatome mit gleichen oder unterschiedlichen Alkylresten und/oder Arylresten und/oder Arylkylresten substituiert werden können, welche hier verallgemeinernd durch die Reste R₉ - R₁₀ dargestellt sind (will sagen, daß die Anzahl der unterschiedlichen Reste nicht notwendig auf bis zu 2 beschränkt ist). a kann dabei vorteilhaft Werte von 1 - 5.000 annehmen.

Im Falle des Einsatzes der siliciumhaltigen Komponenten zur Endverkappung der Polyester erweisen sich unter anderen Verbindungen der Struktur

[[(CH₃)₃Si-O]₂Si[OCH₃]]-O-[R⁷-O]_{q}-[R⁸-O]ᵣ-H

als geeignet, wobei die Reste R⁷ und R⁸ Alkylreste symbolisieren, die gleich oder verschieden sein können mit einer Kohlenstoffanzahl von C₂ bis C₂₂. Zudem gilt für die Koeffizienten: q+r ≥ 0.

Zur Herstellung der erfindungsgemäßen Polyester werden die zur Ausbildung der Seitenkette eingesetzten Alkohole und Säuren bzw Ester vorteilhaft in den molaren Verhältnissen von 1:1 bis etwa 10:1 (1 bzw. 10 Teile Di- oder Polyol) eingesetzt und der sich bildende Alkohol und Wasser und die Überschußkomponente nach erfolgter Kondensation destillativ entfernt. Im Zielmolekül liegen Alkohol- und Säurekomponenten vorzugsweise im ungefähren stöchiometrischen Verhältnis 1:1 vor.

Der Anteil der sulfonsäureresthaltigen Säurekomponenten beträgt 1 bis 99 mol.-%, bevorzugt 10 bis 40 besonders bevorzugt 15 bis 25 mol.-% bezogen auf die Gesamtmenge an Carbonsäuren.

Sehr günstige anwendungstechnische Eigenschaften haben die sulfongruppenhaltigen Polyester der allgemeinen Formel I, wenn als Diolkomponenten 1,2-Propandiol und/oder Diethylenglycol und/oder Cyclohexandimethanol, als Carbonsäuren Isophthalsäure auch mit, 1,3-Cyclohexandicarbonsäure oder auch mit 2,6-Naphthalindicarbonsäure oder auch mit Adipinsäure und als sulfogruppenhaltige Reste 5-Sulfoisophthalsäure-Natriumsalz, das Natriumsalz der Isethionsäure eingesetzt werden.

Nachfolgend ist ein Ausschnitt aus einem erfindungsgemäßen Kammpolymermolekül aufgeführt, wobei eine Polyacrylsäurekette das Rückgrat des Kammpolymemolekuls bildet. Die Säurefunktionen sind mit Polyolen und/oder Oligosilanolen verestert, welche ihrerseits mit einer Säurefunktion von Isophthalsäuremolekülen verestert sind. Weitere Polyole, von denen sich Strukturelemente dieses Polymermoleküls herleiten sind Pentaerythritol, 1,2-Propandiol, Dimethylpolysilanol. Als sulfonatgruppenhaltiges Agens, von dem sich Strukturelemente des Polymermoleküls herleiten, dient beispielsweise das 5-Sulfoisophthalsäuredialkylester-Na-Salz

Aus Gründen der Reaktionsführung, welche dem Fachmann bekannt sind, herrscht im Zielpolymer keine absolute Einförmigkeit der Substitution vor, vielmehr ist von einer gewissen statistischen Verteilungsbreite der Substaitution auszugehen. Ferner werden bestimmte reaktive Molekülgruppierungen auch zu Vernetzung zweier oder mehrerer Polymerketten zu einem mehr oder weniger komplexen Netzwerk zu beobachten sein, wie es das nachfolgende Molekülschema auch darzustellen versucht.

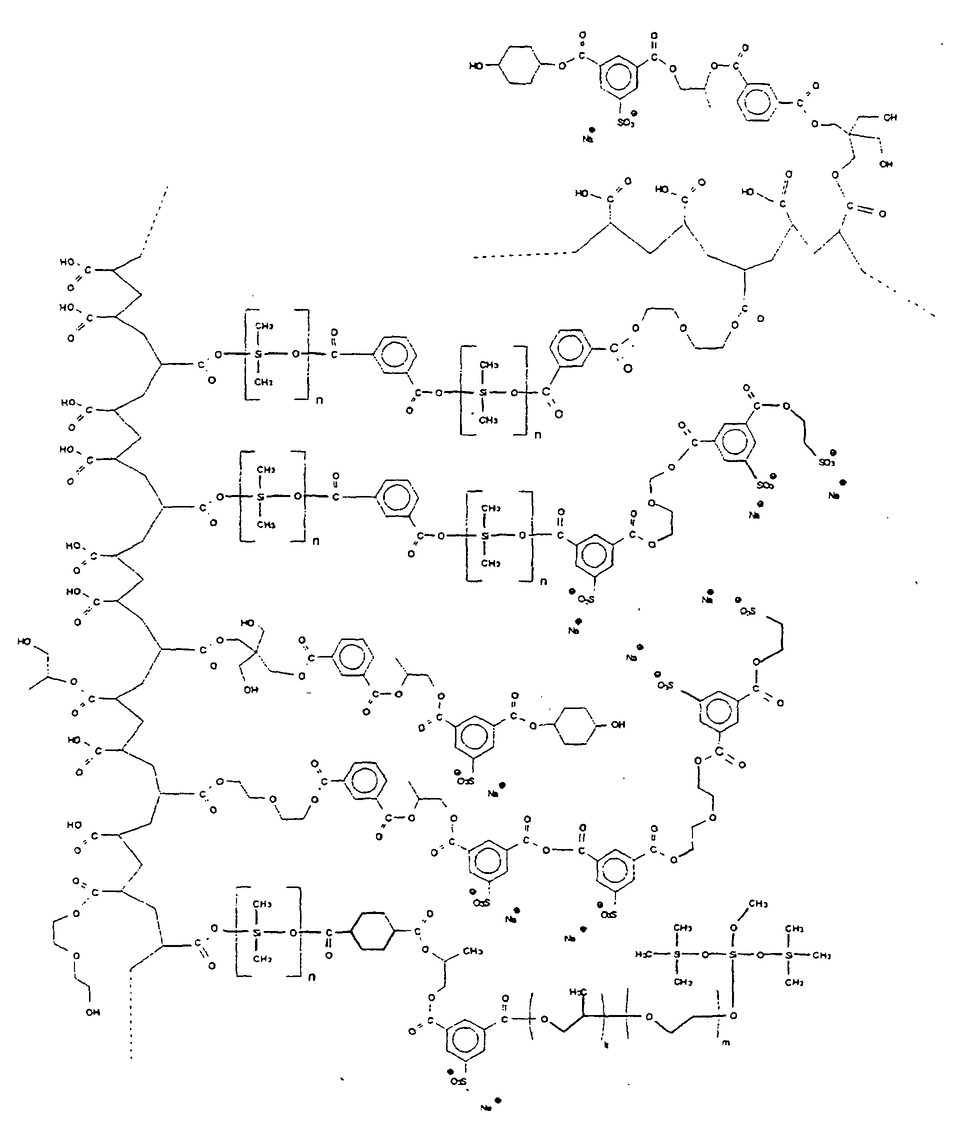

Die erfindungsgemäß einzusetzenden, sulfonhaltigen Polyester sind farblose bis gelbliche, geruchsneutrale Feststoffe. Sie sind in Wasser und Alkoholen gut löslich. Sie können vorteilhaft in kosmetische Zubereitungen zur Festigung der Haare eingearbeitet werden.

Die Herstellung erfindungsgemäßer siliconmodifizierer Kammpolymere erfolgt vorteilhaft, indem ein oder mehrere mehrfunktionelle Alkohole mit einer sulfonsäuregruppenhaltigen, mindestens zwei Carboxylgruppen enthaltenden Substanz, beispielsweise 5-Sulfoisophthalsäuredimethylester-Na-Salz, gegebenenfalls einer weiteren mindestens zwei Carboxylgruppen enthaltenden Substanz und einem Polymer mit einer oder mehreren Polycarbonsäuren, beispielsweise Polyacrylsäure oder Polymethacrylsäure und einem ein- oder mehrfunktionellen Siloxan zusammengegeben, erhitzt und den üblichen Aufbereitungsschritten unterworfen werden.

In einer besonderen Ausführungsform der vorliegenden Erfindung werden die erfindungsgemäßen wasserlöslichen und/oder wasserdispergierbaren Kammpolymeren, bestehend aus einer Polyacrylsäure-enthaltenden Polymerhauptkette und sulfongruppenhaltigen Polyester-Seitenarmen daher in kosmetische, insbesondere haarkosmetische Zubereitungen eingearbeitet.

Zur Anwendung werden die erfindungsgemäßen kosmetischen und dermatologischen Zubereitungen in der für Kosmetika ublichen Weise auf die Haare in ausreichender Menge aufgebracht.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Siliconderivate.

In kosmetischen Zubereitungen zur Festigung der Haare, wie z.B. Haarsprays, Haarlacke, Schaumfestiger, Flüssigfestiger, Stylinggele usw., können die erfindungsgemäß einzusetzenden Kammpolymere vorzugsweise in Konzentrationen von 0.5 bis 30 Gewichtsprozent eingesetzt werden.

Die erfindungsgemäßen Zusammensetzungen zur Festigung der Haare können als Haarsprays oder Schaumaerosole vorliegen und die dafür üblichen und dem Stand der Technik entsprechenden Zusätze enthalten, sofern eine entsprechende Kompatibilität vorliegt. Dies sind beispielsweise weitere Lösungsmittel wie niedere Polyalkohole und deren toxikologisch verträglichen Ether und Ester, Weichmacher, leicht- und schwerflüchtige Silicone, leicht- und schwerflüchtige verzweigte bzw. unverzweigte Kohlenwasserstoffe, Emulgatoren, Antioxidantien, Wachse, Stabilisatoren, pH-Wert-Regulatoren, Farbstoffe, Konsistenzgeber, Antistatika, UV-Absorber, Parfums, usw.

Soll die erfindungsgemäße Zusammensetzung als Haarspray oder Schaumaerosol verwendet werden, so wird in der Regel ein Treibmittel zugesetzt. Übliche Treibmittel sind niedere Alkane, beispielsweise Propan, Butan oder Isobutan, Dimethylether, Stickstoff, Stickstoffdioxid oder Kohlendioxid oder Gemische aus diesen Substanzen.

Bei Verwendung in mechanischen Sprüh- oder Schaumvorrichtungen, beispielsweise Sprühpumpen oder manuellen Schaumpumpen bzw. Squeeze-systemen, kann das Treibmittel in der Regel entfallen.

Die wäßrigen erfindungsgemäßen Zubereitungen enthalten gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrükkenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0.2 - 1.0 % eines Geliermittels) und dabei durchaus feste Konsistenz besitzen. Der Wasseranteil liegt dabei in eisähnlichen Strukturelementen vor, so daß Gele daher ihrer Namensherkunft [aus lat. "gelatum" = "Gefrorenes" über den alchimistischen Ausdruck "gelatina" (16. Jhdt.) für nhdt. "Gelatine"] durchaus gerecht werden.

Gele gemäß der Erfindung enthalten üblicherweise Alkohole niedriger C-Zahl, z.B. Ethanol, Isopropanol, 1,2-Propandiol, Glycerin und Wasser in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Bei kosmetischen und dermatologischen Zubereitungen gemäß der Erfindung beispielsweise kann es sich beispielsweise auch um Shampoonierungsmittel, Zubereitungen zum Fönen oder Einlegen der Haare, Zubereitungen zum Färben, um eine Frisieroder Behandlungslotion handeln.

Erfindungsgemäße Zubereitungen können sich gegebenenfalls vorteilhaft durch einen Gehalt an Tensiden auszeichnen. Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet In wäßriger Lösung bilden sie im sauren oder neutralen Milieu positiv geladene organische Ionen. Amphotere Tenside enthalten sowohl anionische als auch kationische Gruppen und verhalten sich demnach in waßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wäßrigem Medium keine lonen.

### A. Anionische Tenside

Vorteilhaft zu verwendende anionische Tenside sind
Acylaminosäuren (und deren Salze), wie
   1. Acylglutamate, beispielsweise Natriumacylglutamat, Di-TEA-palmitoylaspartat und Natrium Caprylic/ Capric Glutamat,
   2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
   3. Sarcosinate, beispielsweise Myristoyl Sarcosin, TEA-lauroyl Sarcosinat, Natriumlauroylsarcosinat und Natriumcocoylsarkosinat,
   4. Taurate, beispielsweise Natriumlauroyltaurat und Natriummethylcocoyltaurat,
   5. AcylLactylate, lauroyllactylat, Caproyllactylat
   6. Alaninate
Carbonsäuren und Derivate, wie
   1. Carbonsäuren, beispielsweise Laurinsäure, Aluminiumstearat, Magnesiumalkanolat und Zinkundecylenat,
   2. Ester-Carbonsäuren, beispielsweise Calciumstearoyllactylat, Laureth-6 Citrat und Natrium PEG-4 Lauramidcarboxylat,
   3. Ether-Carbonsäuren, beispielsweise Natriumlaureth-13 Carboxylat und Natrium PEG-6 Cocamide Carboxylat,
Phosphorsäureester und Salze, wie beispielsweise DEA-Oleth-10-Phosphat und Dilaureth-4 Phosphat,
Sulfonsäuren und Salze, wie
   1. Acyl-isethionate, z.B. Natrium-/ Ammoniumcocoyl-isethionat,
   2. Alkylarylsulfonate,
   3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
   4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
   sowie
Schwefelsäureester, wie
   1. Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
   2. Alkylsulfate, beispielsweise Natrium-, Ammonium- und TEA- Laurylsulfat.

### B. Kationische Tenside

Gegebenenfalls vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide. Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

In der Regel ist im Sinne der vorliegenden Erfindung die Verwendung von anionischen, amphoteren und/oder nicht-ionischen Tensiden gegenüber der Verwendung von kationischen Tensiden bevorzugt.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Shampoonierungsmittel darstellen, enthalten vorzugsweise mindestens eine anionische nicht-ionische oder amphotere oberflächenaktive Substanz, oder auch Gemische aus solchen Substanzen im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält die erfindungsgemäßen Kammpolymere.

Die erfindungsgemäßen Zusammensetzungen enthalten gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzerextrakte, Vitamine, Wirkstoffe und dergleichen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### (A) Herstellungsbeispiele

### Beispiel 1:

### Reaktionsführung:

In einem 2-lVierhalskolben mit KPG-Rührer, Innenthermometer, Gaseinleitungsrohr und Destillierbrücke werden 1,2-Propandiol, Diethylenglykol und Titantetraisopropylat vorgelegt, kurz verrührt und anschließend Natriumcarbonat und 5-Sulfoisophthalsäuredimethylester-Na-Salz, Isophtalsäure, das entsprechende Siloxan-Diol (Mₙ etwa 4000 g/mol-Aldrich) und Polyacrylsäure eingetragen. Danach wird zweimal evakuiert und mit N₂ inertisiert. Unter Rühren wird nun innerhalb von 30min auf 170 °C erhitzt. Bei ca. 173 °C beginnt die Umesterung bzw. Destillation. Im Laufe von 2 Stunden wird die Innentemperatur auf 210 °C gesteigert. Danach wird die Innentemperatur auf ca. 220 °C gesteigert und weitere 30 min kondensiert. Im Anschluß daran wird in 30 min der Druck auf bestes Vakuum reduziert und 1 Stunde bei 220 °C kondensiert. Anschließend wird mit N₂ belüftet und die Schmelze ausgetragen.

| **Rohstoff** | **Masse (g)** | **Bemerkungen** |
|---|---|---|
| Isophthalsäure | 282,42 | |
| 5-Sulfoisophthalsäure Na-Salz | 88,80 | |
| Polysiloxan-Diol | 40,00 | ca. 10 mmol |
| Polyacrylsäure | 3,00 | |
| Natriumcarbonat | 0,60 | |
| Titantetraisopropylat | 0,60 | |
| 1,2-Propandiol | 104,62 | |
| Diethylenglykol | 119,25 | |
| *2 mol-OH-Gruppen, M=25000 g/mol äquim. COOH Gruppe | | |

### Beispiel 2:

### Reaktionsführung:

In einem 2-l Vierhalskolben mit KPG-Rührer, Innenthermometer, Gaseinleitungsrohr und Destillierbrücke werden 1,2-Propandiol, Diethylenglykol und Titantetraisopropylat vorgelegt, kurz verrührt und anschließend Natriumcarbonat und 5-Sulfoisophthalsäuredimethylester-Na-Salz, Isophtalsäure, Polyacrylsäure und das entsprechende Silicon (Viskosität 1800-2200 Centistokes - Aldrich) eingetragen. Danach wird zweimal evakuiert und mit N₂ inertisiert. Unter Rühren wird nun innerhalb von 30min auf 170 °C erhitzt. Bei ca. 173 °C beginnt die Umesterung bzw. Destillation. Im Laufe von 2 Stunden wird die Innentemperatur auf 210 °C gesteigert. Danach wird die Innentemperatur auf ca. 220 °C gesteigert und weitere 30 min kondensiert. Im Anschluß daran wird in 30 min der Druck auf bestes Vakuum (<1mbar) reduziert und 1 Stunde bei 220 °C kondensiert. Anschließend wird mit N₂ belüftet und die Schmelze ausgetragen.

| **Rohstoff** | **Masse (g)** | **Bemerkungen** |
|---|---|---|
| Isophthalsäure | 286,35 | |
| 5-Sulfoisophthalsäure Na-Salz | 81,40 | |
| Polyacrylsäure* | 5,00 | |
| Dimethylsiloxandiol | 3,00 | (Viskosität 2.000 cSt) |
| Natriumcarbonat | 0,60 | |
| Titantetraisopropylat | 0,60 | |
| 1,2-Propandiol | 195,40 | |
| Diethylenglykol | 222,64 | |

| | | |
|---|---|---|
| *2 mol-OH-Gruppen, M=25000 g/mol äquim. COOH Gruppe | | |

### Beispiel 3:

### Reaktionsführung:

In einem 2-l Vierhalskolben mit KPG-Rührer, Innenthermometer, Gaseinleitungsrohr und Destillierbrücke werden 1,2-Propandiol, Diethylenglykol, Natriumisethionat, 5-Sulphoisophthalsäure-Na-Salz und Titantetraisopropylat vorgelegt, kurz verrührt und anschließend Natriumcarbonat, Isophtalsäure, Polyacrylsäure und das entsprechende Silicon (Viskosität 1800-2200 Centistokes - Aldrich) eingetragen. Danach wird zweimal evakuiert und mit N₂ inertisiert. Unter Rühren wird nun innerhalb von 30 min auf 170 °C erhitzt. Bei ca. 173 °C beginnt die Umesterung bzw. Destillation. Im Laufe von 2 Stunden wird die Innentemperatur auf 210 °C gesteigert. Danach wird die Innentemperatur auf ca. 220 °C gesteigert und weitere 30 min kondensiert. Im Anschluß daran wird in 30 min der Druck auf bestes Vakuum reduziert. Anschließend wird mit N₂ belüftet und die Schmelze ausgetragen.

| **Rohstoff** | **Masse (g)** | **Bemerkungen** |
|---|---|---|
| Isophthalsäure | 290,50 | |
| 5-Sulfoisophthalsäure Na-Salz | 73,45 | |
| Polyacrylsäure* | 4,00 | |
| Natriumisethionat | 8,74 | |
| Dimethylsiloxandiol | 6,00 | (Viskosität 1.800 - 2.200 cST) |
| Natriumcarbonat | 0,60 | |
| Titantetraisopropylat | 0,60 | |
| 1,2-Propandiol | 195,40 | |
| Diethylenglykol | 222,64 | |

| | | |
|---|---|---|
| *2 mol-OH-Gruppen, M=25000 g/mol äquim. COOH Gruppe | | |

### Beispiel 4:

### Reaktionsführung:

In einem 2-l Vierhalskolben mit KPG-Rührer, Innenthermometer, Gaseinleitungsrohr und Destillierbrücke werden 1,2-Propandiol, Diethylenglykol, Natriumisethionat, 1,4-Cyclohexandicarbonsäure und Titantetraisopropylat vorgelegt, kurz verrührt und anschließend Natriumcarbonat und 5-Sulfoisophthalsäuredimethylester-Na-Salz, Isophtalsäure das entsprechende Silicon (Silvet 867 - WITCO (Propoxyliertes Trisiloxan - monofunktionell bezüglich der reaktiven Gruppe, in diesem Fall OH)) und Polyacrylsäure eingetragen. Danach wird zweimal evakuiert und mit N₂ inertisiert. Unter Rühren wird nun innerhalb von 30min auf 170 °C erhitzt. Bei ca. 173 °C beginnt die Umesterung bzw. Destillation. Im Laufe von 2 Stunden wird die Innentemperatur auf 210 °C gesteigert. Danach wird die Innentemperatur auf ca. 220 °C gesteigert und weitere 30 min kondensiert. Im Anschluß daran wird in 30 min der Druck auf bestes Vakuum reduziert und 1 Stunde bei 220 °C kondensiert. Anschließend wird mit N₂ belüftet und die Schmelze ausgetragen.

| **Rohstoff** | **Masse (g)** |
|---|---|
| Isophthalsäure | 66,45 |
| 1,4-Cyclohexandicarbonsäure | 199,20 |
| Natriumisethionat | 14,21 |
| 5-Sulfoisophthalsäure Na-Salz | 118,49 |
| Polyacrylsäure* | 3,00 |
| Natriumcarbonat | 0,60 |
| Titantetraisopropylat | 0,60 |
| Silvet-867 | 6,00 |
| 1,2-Propandiol | 195,40 |
| Diethylenglykol | 166,95 |

| | |
|---|---|
| *2 mol-OH-Gruppen, M=25000 g/mol äquim. COOH Gruppe | |

### Beispiel 5:

### Reaktionsführung:

In einem 2-l Vierhalskolben mit KPG-Rührer, Innenthermometer, Gaseinleitungsrohr und Destillierbrücke werden 1,2-Propandiol, Diethylenglykol, Natriumisethionat und Titantetraisopropylat vorgelegt, kurz verrührt und anschließend Natriumcarbonat und 5-Sulfoisophthalsäure-Li-Salz, Isophtalsäure, 1,4-Cyclohexandicarbonsäure und das entsprechende Silicon (Dimeres von Dimethyldihydroxysilanol, entspricht einem beidseitig OH-endfunktionalisierten Polydimethylsiloxan) und Polyacrylsäure eingetragen. Danach wird zweimal evakuiert und mit N₂ inertisiert. Unter Rühren wird nun innerhalb von 30 min auf 170 °C erhitzt. Bei ca. 173 °C beginnt die Umesterung bzw. Destillation. Im Laufe von 2 Stunden wird die Innentemperatur auf 210 °C gesteigert. Danach wird die Innentemperatur auf ca. 220 °C gesteigert und weitere 30 min kondensiert. Im Anschluß daran wird in 30 min der Druck auf bestes Vakuum (< 1mbar) reduziert und 1 Stunde bei 220 °C kondensiert. Anschließend wird mit N₂ belüftet und die Schmelze ausgetragen.

| **Rohstoff** | **Masse (g)** |
|---|---|
| Isophthalsäure | 66,92 |
| Disiloxan (OH-funktionalisiert) | 5,15 |
| Natriumisethionat | 10,94 |
| 1,4-Cyclohexandicarbonsäure | 206,62 |
| 5-Sulfoisophthalsäure Li-Salz | 113,45 |
| Polyacrylsäure* | 3,00 |
| Natriumcarbonat | 0,60 |
| Titantetraisopropylat | 0,60 |
| Diethylenglycol | 53,10 |
| 1,2-Propandiol | 152,18 |

| | |
|---|---|
| *2 mol-OH-Gruppen, M=25000 g/mol äquim. COOH Gruppe | |

### Beispiel 6:

### Reaktionsführung:

In einem 2-l Vierhalskolben mit KPG-Rührer, Innenthermometer, Gaseinleitungsrohr und Destillierbrücke werden 1,4-Cyclohexandimethanol, 1,4-Cyclohexandicarbonsäure, 1,2-Propandiol, Diethylenglykol, Natriumisethionat und Titantetraisopropylat vorgelegt, kurz verrührt und anschließend Natriumcarbonat, 5-Sulfoisophthalsäure-Li-Salz ("LI-SIM"), 5-Sulfoisophthalsäure-dimetylester-Na-Salz ("NA-SIM"), Isophtalsäure, Pentaerythrit, das entsprechende Silicon (Dimeres von Dimethyldihydroxysilanol, entspricht einem beidseitig OH-endfunktionalisierten Polydimethylsiloxan) und Polyacrylsäure eingetragen. Danach wird zweimal evakuiert und mit N₂ inertisiert. Unter Rühren wird nun innerhalb von 30 min auf 170 °C erhitzt. Bei ca. 173 °C beginnt die Umesterung bzw. Destillation. Im Laufe von 2 Stunden wird die Innentemperatur auf 210 °C gesteigert. Danach wird die Innentemperatur auf ca. 220 °C gesteigert und weitere 30 min kondensiert. Im Anschluß daran wird in 30 min der Druck auf bestes Vakuum (< 1 mbar) reduziert und 1 Stunde bei 220 °C kondensiert. Anschließend wird mit N₂ belüftet und die Schmelze ausgetragen.

| **Rohstoff** | **Masse (g)** |
|---|---|
| Isophthalsäure | 66,92 |
| Natriumisethionat | 10,94 |
| 1,4-Cyclohexandicarbonsäure | 206,62 |
| 1,4-Cyclohexandimethanol | 57,68 |
| Li-SIM | 75,64 |
| Na-SIM | 44,43 |
| Polyacrylsäure* | 3,00 |
| Disiloxan (OH-funktionalisiert) | 1,21 |
| Natriumcarbonat | 0,60 |
| Titantetraisopropylat | 0,60 |
| Pentaerythrit | 6,81 |
| Diethylenglycol | 53,10 |
| 1,2-Propandiol | 152,18 |

| | |
|---|---|
| *2 mol-OH-Gruppen, M=25000 g/mol äquim. COOH Gruppe | |

### (B) Rezepturbeispiele:

### Haarsprays: Beispiele 1 - 6

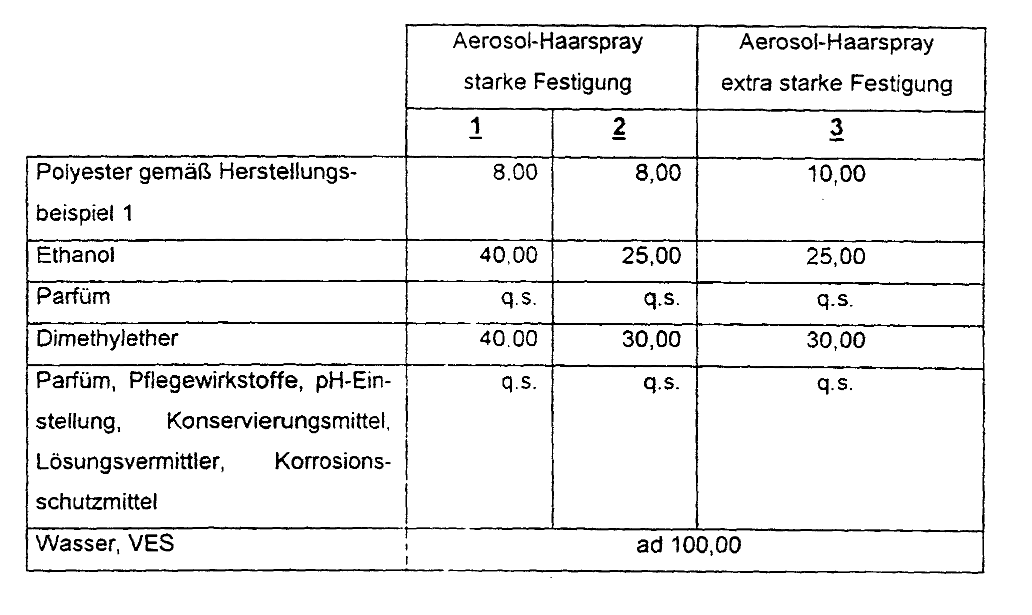

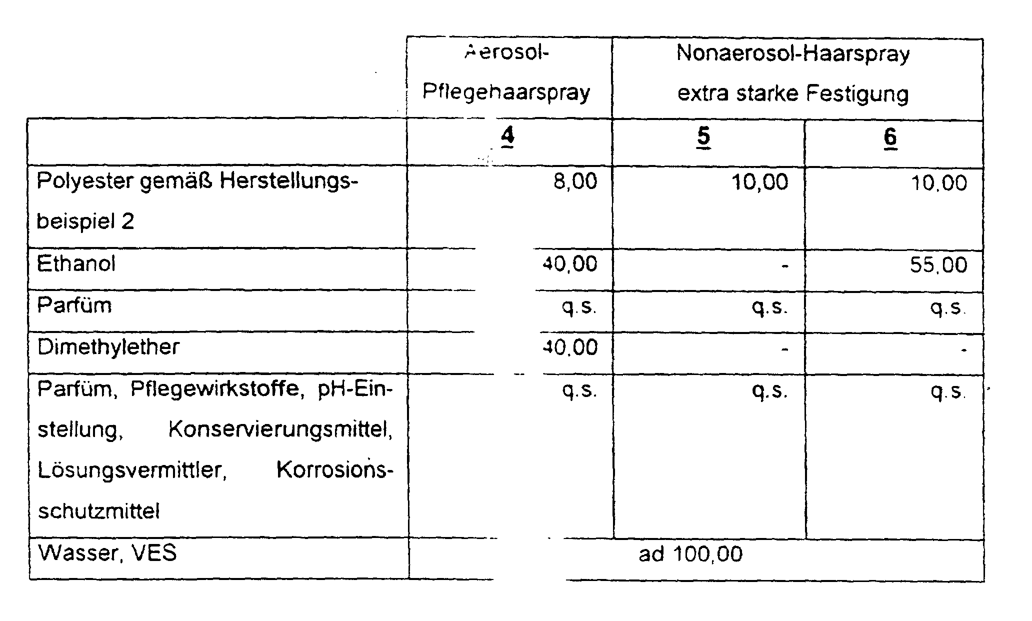

### Schaumfestiger: Beispiele 7 - 8

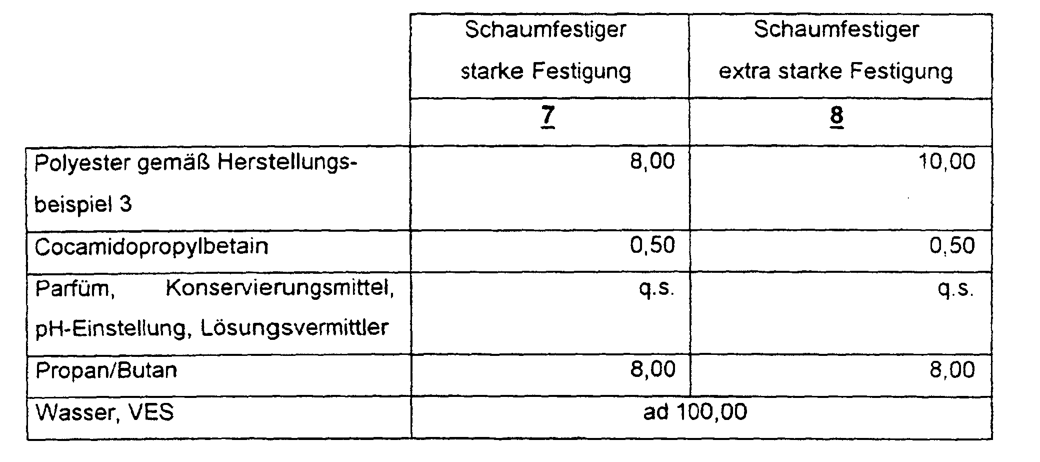

### Stylinggele: Beispiele 9 - 10

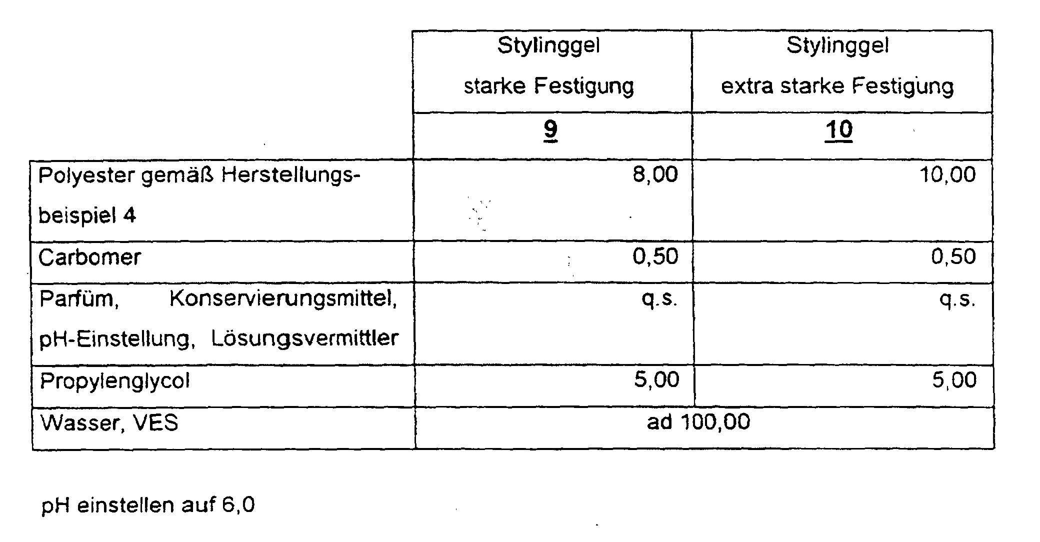

### Styling-Shampoos: Beispiel 11 - 12

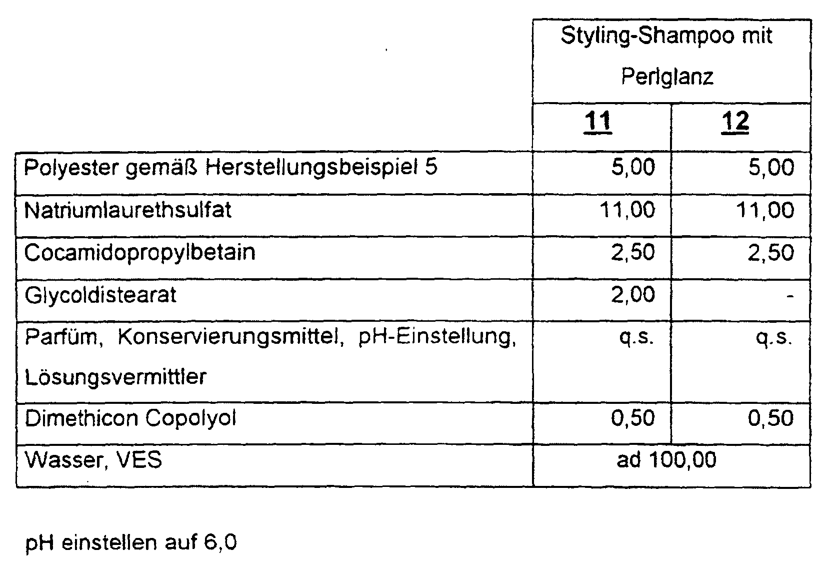

## Patentansprüche

1. Haarbehandlungsmittel mit einem wirksamen Gehalt an einem oder mehreren wasserlöslichen und/oder wasserdispergierbare siliconmodifizierte Kammpolymeren, welche gewählt werden aus der Gruppe der Polyester folgender generischer Strukturformeln 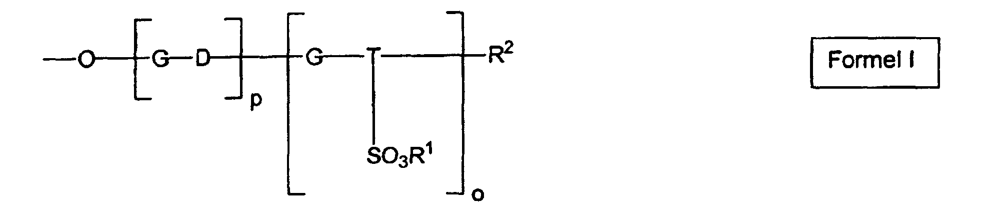 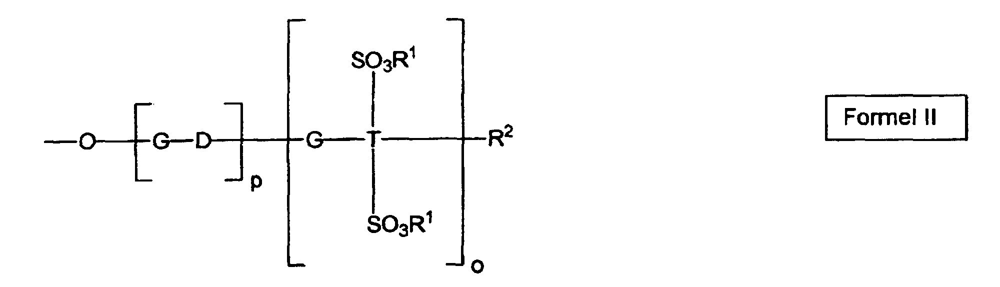 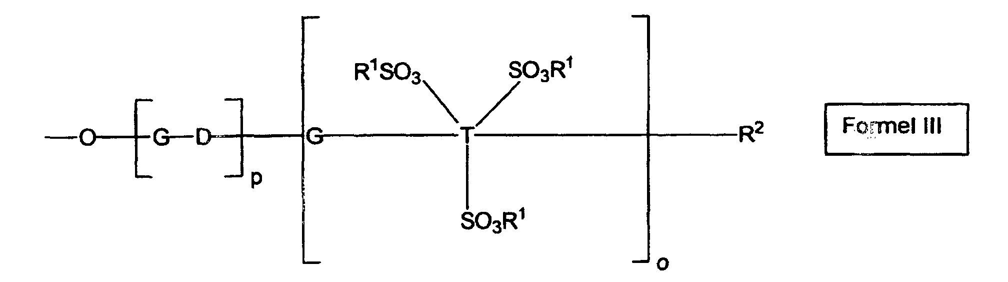 usw.
wobei p und m so gewählt werden, dass mittlere Molekulargewichte der eingesetzten Hauptkettenbestandteile zwischen 200 und 2.000.000 g/mol liegen, wobei der Bereich von 2.000 -100.000 g/mol bevorzugt Verwendung findet,
die Polyester-Seitenketten gemäß Formel I - III bestehen aus:
G: einer mindestens zwei endständige Sauerstoffatome enthaltende Siloxaneinheit, die vorteilhaft durch Strukturelemente charakterisiert ist wie folgt: 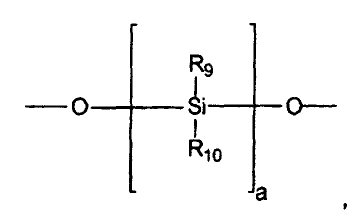 wobei die Siliciumatome mit gleichen oder unterschiedlichen Alkylresten und/oder Arylresten und/oder Arylakylresten substituiert werden können, welche hier verallgemeinernd durch die Reste R₉ - R₁₀ dargestellt sind (will sagen, dass die Anzahl der unterschiedlichen Reste nicht notwendig auf bis zu 2 beschränkt ist). a kann dabei vorteilhaft Werte von 1 - 5.000 annehmen,
sowie der mindestens zwei endständige Sauerstoffatome enthaltende aromatischen, aliphatischen oder cycloaliphatischen Organyleinheiten mit einer Kohlenstoffzahl von C₂ bis C₂₂ oder Abkömmlinge eines Polyglykols der Form HO-[R³-O)ₖ-[R⁴-O)ₘ-H, entsprechend einer Organyleinheit 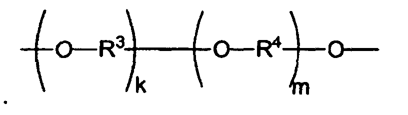 wobei die Reste R³ und R⁴ Alkylenreste darstellen mit einer Kohlenstoffzahl von C₂-C_{22,} wobei beide Reste nicht notwendigerwiese verschieden sein müssen.
wobei für die Koëffzienten k und m gilt: k+m ≥ 1, wobei k und m ferner so gewählt werden können, dass die vorab bezeichneten mittleren Molekulargewichte der eingesetzen Hauptkettenbestandteile zuwege kommen;
D: einer mindestens zwei endständige Acylgruppen entahltenden aromatischen, aliphatischen oder cycloaliphatischen Organyleinheit mit einer Kohlenstoffzahl von C₂ bis C₂₂, wobei auch Kombinationen aus mehreren verschiedenen Säurekomponenten im beanspruchten Zielmolekül enthalten sein können, beispielsweise eine Organyleinheit des Schemas 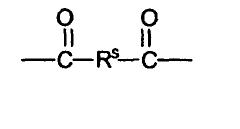
T: eine Verbindung aus der Gruppe der mindestens zwei endständige Acylgruppen enthaltenden sulfonierten aromatischen, aliphatischen oder cycloaliphatischen Organylverbindungen
R¹: Lithium, Natrium, Kalium, Magnesium, Calcium, Ammonium, Monoalkylammonium, Dialkylammonium, Trialkylammonium oder Tetraalkylammonium bedeuten kann, worin die Alkylpositionen der Amine unabhängig voneinander mit C₁ bis C₂₂-Alkylresten und 0 bis 3 Hydroxylgruppen besetzt sind;
R²: einen Molekülrest, gewählt aus den Gruppen der
- über Etherfunktionen verbrückenden monofunktionell-linearen oder -verzweigten siliconhaltigen Organylreste,
- aromatischen, aliphatischen oder cycloaliphatischen Aminofunktionen: (-NH-R⁵, -NR⁵₂ wobei R⁵ einen Alkyl- oder Arylrest mit C₁ bis C₂₂ darstellen kann)
- aromatischen, aliphatischen oder cycloaliphatischen Monocarbonsäuregruppen: (-COOR⁶ wobei R⁶ ein Alkyl- oder Arylrest darstellt mit C₁ bis C₂₀₀)
- über Etherfunktionen verbrückten aromatischen, aliphatischen oder cycloaliphatischen Organylreste: (-O-R⁵)
- über Etherfunktionen verbrückenden Polyalkoxyverbindungen der Form
-O-[R⁷-O]_{q}-[R⁸-O]ᵣ-Y
Die Reste R⁷ und R⁸ stellen vorteilhaft Alkylenreste dar mit einer Kohlenstoffzahl von C₂-C₂₂, wobei beide Reste nicht notwendigerweise verschieden sein müssen. Der Rest Y kann sowohl Wasserstoff als auch aliphatischer Natur mit C₁-C₂₂ sein. Für die Koeffizienten q und r gilt: q+r ≥ 1.
- über Etherfunktionen verbrückenden einfach oder mehrfach ethoxylierten sulfonierten Organylreste oder bevorzugt deren Alkali- oder Erdalkalisalze, wie beispielsweise vorteilhaft **gekennzeichnet durch** die generische Strukturformel
-(O-CH₂-CH₂)ₛ-SO₃R¹
mit s ≥ 1, und wobei s ferner so gewählt werden kann, dass die vorab bezeichneten mittleren Molekulargewichte der eingesetzen Hauptkettenbestandteile zuwege kommen.
- Siliconfunktionen, die sich von monofunktionalen Siliconen ableiten gemäß der generischen Struktformel 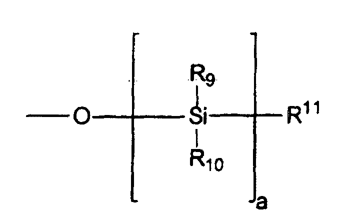
wobei R⁹ und R¹⁰ die genannten Eigenschaften haben, und unabhängi davon R¹¹ ebenfalls einen Alkylrest oder einen Arylreste oder einen Aryalkylrest darstellen kann.

2. Haarbehandlungsmittel nach Anspruch 1, **dadurch gekennzeichnet, dass** der Siliciumanteil in den Gruppierungen 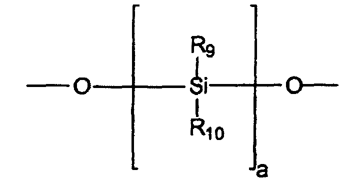 und/oder 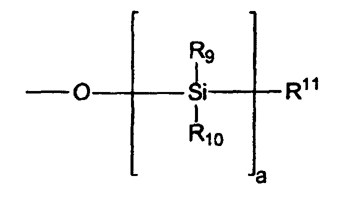 zwischen 0,1 und 50 mol-% liegt, wobei die mittleren Molekulargewichte bevorzugt zwischen 100 und 100.000 g/mol liegen, wobei der Bereich für monofunktionelle siliconhaltige Derivate zwischen 100 und 2.000 g/mol bzw. für mindestens difunktionelle siliconhaltige Derivate zwischen 100 und 30.000 g/mol besonders bevorzugt wird.

3. Haarbehandlungsmittel nach Anspruch 1, **dadurch gekennzeichnet, dass** die mittleren Molekulargewichte der Kammpolymeren zwischen 200 und 2.000.000 g/mol liegen, besonders vorteilhaft zwischen 200 und 100.000 g/mol, wobei der Bereich von 1.000 - 30.000 g/mol bevorzugt Verwendung findet, ganz besonders vorteilhaft von 5.000 - 15.000 g/mol.

## Claims

1. Hair-treatment compositions with an effective content of one or more water-soluble and/or water-dispersible silicone-modified comb polymers which are chosen from the group of polyesters of the following generic structural formulae 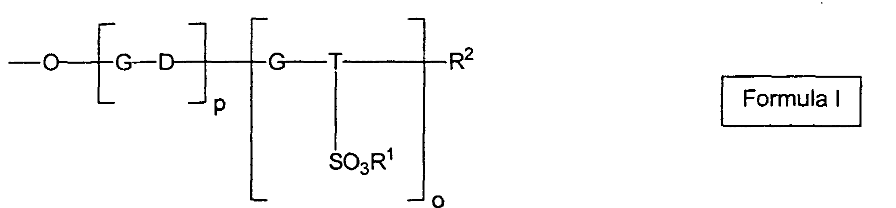 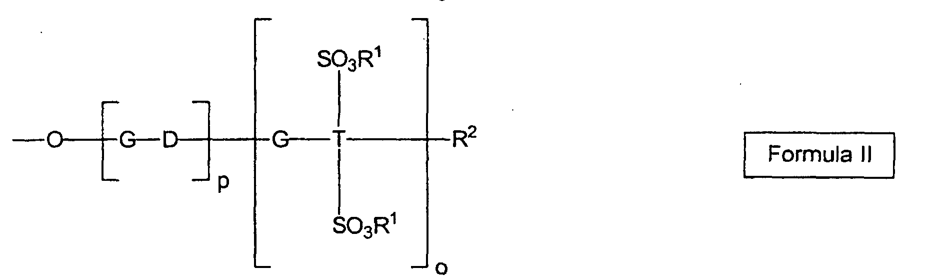 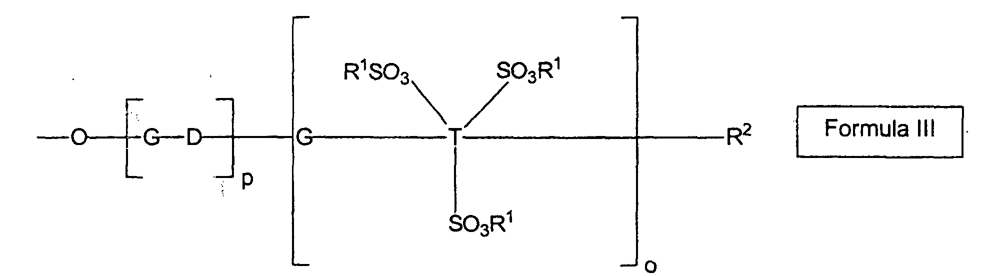 where p and o are chosen such that average molecular weights of the main chain constituents used are between 200 and 2,000,000 g/mol, where the range 2000 - 100,000 g/mol is preferably used,
the polyester side chains according to formula I - III consist of:
G : a siloxane unit containing at least two terminal oxygen atoms which is advantageously **characterized by** structural elements as follows: 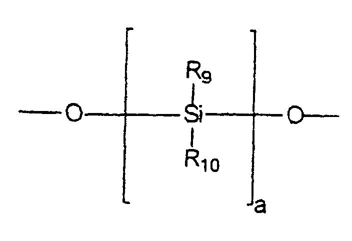 where the silicon atoms can be substituted by identical or different alkyl radicals and/or aryl radicals and/or arylalkyl radicals, which are shown here by way of generalization by the radicals R₉ - R₁₀ (it should be said that the number of different radicals is not necessarily limited to 2). a can here advantageously assume values of 1 - 5,000,
and the aromatic, aliphatic or cycloaliphatic organyl units containing at least two terminal oxygen atoms and having a carbon number from C₂ to C₂₂ or derivatives of a polyglycol of the form HO-[R³-O]ₖ-[R⁴-O]ₘ-H, corresponding to an organyl unit 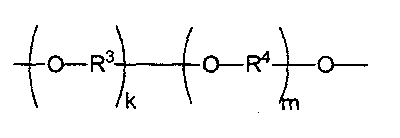 where the radicals R³ and R⁴ are alkylene radicals having a carbon number of from C₂-C₂₂, where the two radicals do not necessarily have to be different,
where the following applies for the coefficients k and m: k+m ≥ 1, where k and m can also be chosen such that the average molecular weights, referred to previously, of the main chain constituents used are achieved;
D : an aromatic, aliphatic or cycloaliphatic organyl unit having a carbon number of from C₂ to C₂₂ and containing at least two terminal acyl groups, where combinations of two or more different acid components may also be present in the claimed target molecule, for example an organyl unit of the scheme 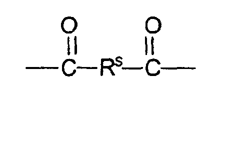
T : a compound from the group of the sulphonated aromatic, aliphatic or cycloaliphatic organyl compounds containing at least two terminal acyl groups
R¹: can be lithium, sodium, potassium, magnesium, calcium, ammonium, monoalkylammonium, dialkylammonium, trialkylammonium or tetraalkylammonium, in which the alkyl positions of the amines are, independently of one another, occupied by C₁ to C₂₂-alkyl radicals and 0 to 3 hydroxyl groups;
R²: a molecular moiety chosen from the groups of
- monofunctionally linear or branched s ilicon-containing o rganyl radicals b ridging via ether functions,
- aromatic, aliphatic or cycloaliphatic amino functions: ( -NH-R⁵, -NR⁵₂, where R⁵ can be an alkyl or aryl radical with C, to C₂₂)
- aromatic, aliphatic or cycloaliphatic monocarboxylic acid groups: (-COOR⁶, where R⁶ is an alkyl or aryl radical with C, to C₂₀₀)
- aromatic, aliphatic or cycloaliphatic organyl radicals bridged via ether functions: (-O-R⁵)
- polyalkoxy compounds bridging via ether functions and of the form
-O-[R⁷-O]_{q}-[R⁸-O]ᵣ-Y
The radicals R⁷ and R⁸ are advantageously alkyl radicals having a carbon number of from C₂ to C₂₂, where the two radicals do not necessarily have to be different. The radical Y can either be hydrogen or of an aliphatic nature with C₁-C₂₂. For the coefficients q and r the following applies: q+r ≥ 1.
- mono- or polyethoxylated sulphonated organyl radicals bridging via ether functions, or preferably alkali metal or alkaline earth metal salts thereof, such as, for example, advantageously **characterized by** the generic structural formula
-(O-CH₂-CH₂)ₛ-SO₃R¹
where s ≥ 1, and where s can also be chosen such that the average molecular weights, referred to previously, of the main chain constituents used are achieved.
- silicone functions derived from monofunctional silicones according to the generic structural formula 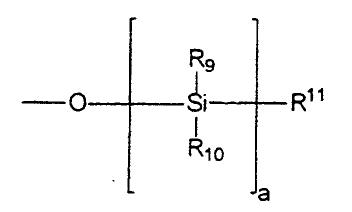
where R₉ and R₁₀ have said properties and, independently thereof, R₁₁ can likewise represent an alkyl radical or an aryl radical or an arylalkyl radical.

2. Hair-treatment compositions according to Claim 1, **characterized in that** the silicon content in the groups 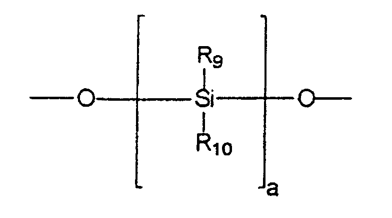 and/or 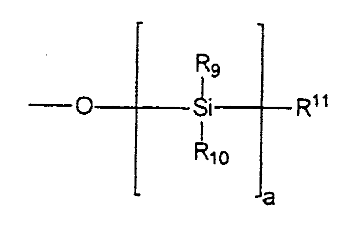 is between 0.1 and 50 mol% where the average molecular weights are preferably between 100 and 100,000 g/mol, where the range for monofunctional silicone-containing derivatives is particularly preferably between 100 and 2000 g/mol or for at least difunctional silicone-containing derivatives between 100 and 30,000 g/mol.

3. Hair-treatment compositions according to Claim 1, **characterized in that** the average molecular weights of the comb polymers are between 200 and 2,000,000 g/mol, particularly advantageously between 200 and 100,000 g/mol, the range 1000 - 30,000 g/mol being preferably used, very particularly advantageously 5000 - 15,000 g/mol.

## Revendications

1. Agents de traitement des cheveux avec une teneur active en un ou plusieurs polymères en peigne, solubles et/ou dispersibles dans l'eau, modifiés par silicone, qui sont choisis dans le groupe des polyesters présentant les formules de structure génériques suivantes : 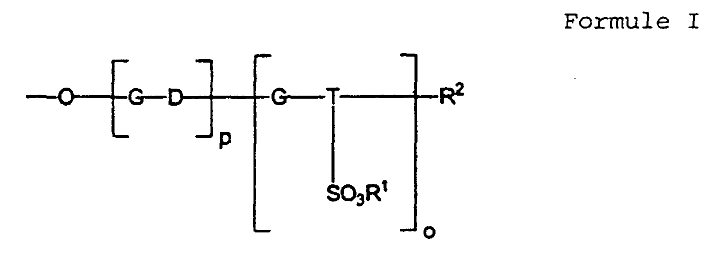 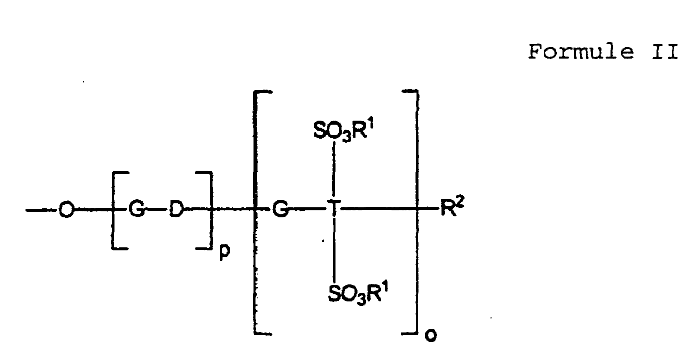 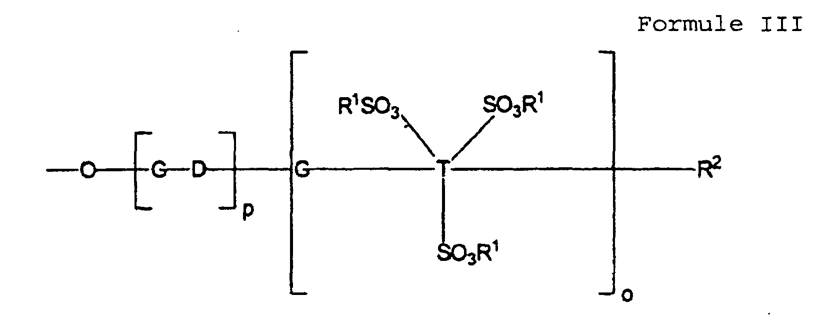 etc.,
p et o étant choisis de telle manière que les poids moléculaires moyens des constituants de chaîne principale utilisés sont situés entre 200 et 2000000 g/mole, la plage de 2000 à 100000 g/mole étant utilisée de préférence,
les chaînes latérales polyester selon les formules I à III étant constituées par
G : une unité siloxane contenant au moins deux atomes d'oxygène en position d'extrémité, qui est avantageusement **caractérisée par** les éléments de structure comme suit : 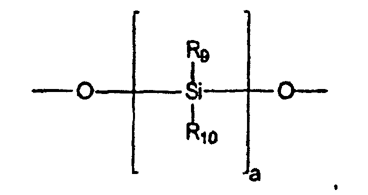 les atomes de silicium pouvant être substitués par des radicaux alkyle et/ou des radicaux aryle et/ou des radicaux arylalkyle identiques ou différents, qui sont représentés ici de manière généralisée par les radicaux R₉-R₁₀ (ce qui signifie que le nombre des différents radicaux n'est pas nécessairement limité à 2), a peut avantageusement valoir 1 à 5000,
ainsi que des unités organyle aromatiques, aliphatiques ou cycloaliphatiques contenant au moins deux atomes d'oxygène en position terminale, présentant un indice de carbone de C₂ à C₂₂ ou des dérivés d'un polyglycol de formule HO[R³-O]ₖ-[R⁴-O]ₘ-H, correspondant à une unité organyle 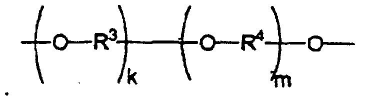 les radicaux R³ et R⁴ représentant des radicaux alkylène avec un indice de carbone de C₂ à C₂₂, les deux radicaux ne devant pas nécessairement être différents,
où, pour les coefficients k et m, la relation suivante est d'application : k+m ≥ 1, k et m pouvant en outre être choisis de telle manière que les poids moléculaires moyens indiqués ci-dessus peuvent être atteints par les constituants de la chaîne principale utilisés,
D : une unité organyle aromatique, aliphatique ou cycloaliphatique contenant au moins deux groupes acyle en position terminale, présentant un indice de carbone de C₂ à C₂₂, des combinaisons de plusieurs composants acides différents pouvant être contenues dans la molécule cible revendiquée, par exemple une unité organyle du schéma 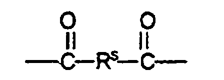 T : un composé du groupe des composés organyle aromatiques, aliphatiques ou cycloaliphatiques sulfonés, contenant au moins deux groupes acyle en position terminale,
R¹ : peut signifier lithium, sodium, potassium, magnésium, calcium, ammonium, monoalkylammonium, dialkylammonium, trialkylammonium ou tétraalkylammonium, les positions alkyle des amines étant occupées indépendamment les unes des autres par des radicaux alkyle en C₁ à C₂₂ et 0 à 3 groupes hydroxyle,
R² : peut représenter un reste de molécule, choisi dans les groupes constitués par
- les radicaux organyle contenant de la silicone, monofonctionnels, linéaires ou ramifiés, formant des ponts via des fonctions éther,
- les fonctions amino aromatiques, aliphatiques ou cycloaliphatiques : (-NH-R⁵, -NR⁵₂, R⁵ pouvant représenter un radical alkyle ou aryle en C₁ à C₂₂)
- les groupes acide monocarboxylique aromatiques, aliphatiques ou cycloaliphatiques : (-COOR⁶, R⁶ représentant un radical alkyle ou aryle en C₁ à C₂₀₀)
- les radicaux organyle aromatiques, aliphatiques ou cycloaliphatiques liés par un pont via des fonctions éther : (-O-R⁵)
- les composés polyalcoxy formant des ponts via des fonctions éther de formule
-O-[R⁷-O]_{q}-[R⁸-O]ᵣ-Y
les radicaux R⁷ et R⁸ représentant avantageusement des radicaux alkylène avec un indice de carbone de C₂ à C₂₂, les deux radicaux ne devant pas nécessairement être différents. Le radical Y peut être un hydrogène ainsi que de nature aliphatique en C₁ à C₂₂. Pour les coefficients q et r, la relation suivante est d'application : q+r ≥ 1,
- les radicaux organyle sulfonés monoéthoxylés ou polyéthoxylés formant un pont via des fonctions éther ou de préférence leurs sels de métal alcalin ou alcalino-terreux, tels que par exemple avantageusement **caractérisés par** la formule de structure générique
-(O-CH₂-CH₂)ₛ-SO₃R¹
avec s ≥ 1 et s étant choisi en outre de telle manière que les poids moléculaires moyens indiqués ci-dessus peuvent être atteints par les constituants de la chaîne principale utilisés,
- les fonctions silicone, qui sont dérivées des silicones monofonctionnelles selon la formule de structure générique 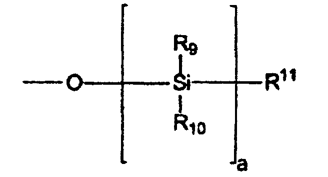
où R⁹ et R¹⁰ présentent les propriétés mentionnées et R¹¹ pouvant également représenter, indépendamment, un radical alkyle ou un radical aryle ou un radical arylalkyle.

2. Agent de traitement de cheveux selon la revendication 1, **caractérisé en ce que** la proportion de silicium dans les groupes 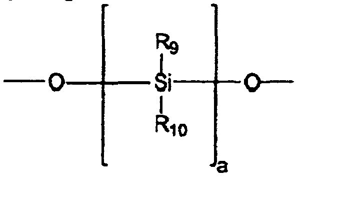 et/ou 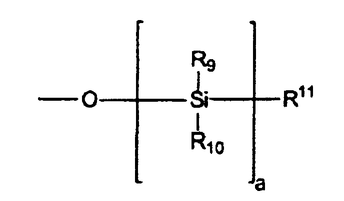 est comprise entre 0,1 et 50% en mole, les poids moléculaires moyens étant de préférence situés entre 100 et 100000 g/mole, la plage entre 100 et 2000 g/mole étant particulièrement préférée pour les dérivés monofonctionnels contenant de la silicone et la plage entre 100 et 30000 g/mole étant particulièrement préférée pour les dérivés- au moins difonctionnels contenant de la silicone.

3. Agent de traitement pour cheveux selon la revendication 1, **caractérisé en ce que** les poids moléculaires moyens des polymères en peigne sont situés entre 200 et 2000000 g/mole, de manière particulièrement avantageuse entre 200 et 100000 g/mole, la plage de 1000 à 300000 g/mole étant utilisée de préférence, de manière tout à fait avantageuse de 5000 à 15000 g/mole.
